(19)

Europäisches
Patentamt
European
Patent Office
Office européen
des brevets

(11) **EP 4 711 792 A1**

(12) **EUROPEAN PATENT APPLICATION**

(43) Date of publication:
**18.03.2026 Bulletin 2026/12**

(21) Application number: **24200222.8**

(22) Date of filing: **13.09.2024**

(51) International Patent Classification (IPC):
**G01R 33/48** $^{(2006.01)}$        **A61B 5/00** $^{(2006.01)}$
**G01R 33/56** $^{(2006.01)}$        G01R 33/54 $^{(2006.01)}$

(52) Cooperative Patent Classification (CPC):
**G01R 33/4828; A61B 5/055; A61B 5/4872;**
**G01R 33/5608;** G01R 33/543

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **EGGERS, Holger**
**Eindhoven (NL)**

(74) Representative: **Philips Intellectual Property &**
**Standards**
**High Tech Campus 52**
**5656 AG Eindhoven (NL)**

(54) **DETERMINATION OF FAT FRACTION IN DIXON MAGNETIC RESONANCE IMAGING**

(57)      Disclosed herein is a method of determining a fat fraction value (130). The method comprises receiving (200) a water Dixon image (122) and a fat Dixon image (124) descriptive of a region of a subject (318). The method further comprises calculating (202) a modified fat fraction map (126) using the water Dixon image and the fat Dixon image, wherein the modified fat fraction map is limited to fat fraction values greater than or equal to zero and preferably less than or equal to one. The method further comprises displaying (204) the modified fat fraction map on a user interface (108) and receiving (206) a selection of a measurement ROI (128) descriptive of an area within the region of the subject from the user interface. The method further comprises calculating (208) the fat fraction value for the measurement ROI using the water Dixon image and the fat Dixon image and providing (210) the fat fraction value. Systems are disclosed, configured to perform the method.

Fig. 2

Processed by Luminess, 75001 PARIS (FR)

**Description**

TECHNICAL FIELD

The invention relates to magnetic resonance imaging, in particular to Dixon magnetic resonance imaging.

BACKGROUND

Dixon MRI is an MRI technique capable of the quantification of tissue composition, particularly in measuring fat fraction. Dixon MRI employs chemical shift imaging to differentiate between water and fat signals within the body. By acquiring images at different echo times, Dixon MRI effectively separates and quantifies the water and fat content in tissues, allowing for precise fat fraction measurement. The determination of the fat fraction is useful in a variety of clinical applications, such as evaluating hepatic steatosis, assessing muscle fat infiltration, and diagnosing other conditions associated with abnormal fat accumulation.

SUMMARY

The invention provides for a method, which may be a computer implemented method, a computer program product, and a medical system in the independent claims. Embodiments are given in the dependent claims.

In one aspect a method of determining a fat fraction value is disclosed. The method comprises receiving a water Dixon image and a fat Dixon image that are both descriptive of a common region of a subject. The method further comprises calculating a modified fat fraction map using the water Dixon image and the fat Dixon image. The modified fat fraction map is limited to fat fraction values greater than or equal to 0 and preferably less than or equal to 1. The method further comprises displaying the modified fat fraction map on a user interface. The method further comprises receiving a selection of a measurement region of interest (ROI) that is descriptive of an area within the common region of the subject from the user interface. The method further comprises calculating a fat fraction value for the measurement ROI using the water Dixon image and the fat Dixon image. The method further comprises providing the fat fraction value.

In another aspect a computer program product comprising machine-executable instructions configured for causing the computational system to perform the method of determining the fat fraction value is disclosed.

In another aspect, a medical system is disclosed. The medical system comprises a computational system configured to: receive a water Dixon image and a fat Dixon image descriptive of a common region of a subject; calculate a modified fat fraction map using the water Dixon image and the fat Dixon image. The modified fat fraction map is limited to a fat fraction value greater than or equal to 0 and preferably less than or equal to 1. The computational system is further configured to display the modified fat fraction map on a user interface; receive a selection of a measurement ROI descriptive of an area within the common region of the subject from the user interface; calculate a fat fraction value for the measurement ROI using the water Dixon image and the fat Dixon image and to provide the fat fraction value.

BRIEF DESCRIPTION OF THE DRAWINGS

In the following preferred embodiments of the invention will be described, by way of example only, and with reference to the drawings in which:

Fig. 1 illustrates an example of a medical system.
Fig. 2 shows a flow chart, which illustrates a method according to the invention.
Fig. 3 illustrates a further example of a medical system.
Fig. 4 shows a flow chart, which illustrates a further method according to the invention.
Fig. 5 illustrates an example of a cloud-based medical system.
Fig. 6 illustrates the effect of noise on the fat fraction value calculated using different methods.
Fig. 7 shows results of similar calculations to those shown in Fig. 6, however the individual fat fraction values were enforced to be positive values.
Fig. 8 depicts a single echo image reconstructed from the Dixon k-space data.
Fig. 9 depicts the water Dixon image 122 and the fat Dixon image 124 from the same Dixon k-space data.
Fig. 10 depicts a modified fat fraction map that was calculated from the Dixon k-space data.
Fig. 11 depicts a pixel wise fat fraction map.
Fig. 12 depicts an example of a user interface.
Fig. 13 depicts an example of an indicator on the user interface.

DESCRIPTION OF EMBODIMENTS

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner. Like numbered elements in these figures are either equivalent elements or perform the same function. Elements which have been discussed previously will not necessarily be discussed in later figures if the function is equivalent.

In an example, there is a computer-implemented method of determining a fat fraction value. The method comprises receiving a water Dixon image and a fat Dixon image that are descriptive of a common region of a subject. Dixon magnetic resonance imaging is a magnetic resonance imaging technique in which the signal from water and fat are separated by acquiring images at different echo times and analyzing the variation in signal amplitude and phase over echo times. By performing Dixon magnetic resonance imaging a so-called water Dixon image and a fat Dixon image can be reconstructed. The fat fraction is the fraction of the signal within a particular voxel which is due to the fat signal. The fat fraction in itself may be extremely useful in diagnosing several diseases such as hepatic steatosis.

The method further comprises calculating a modified fat fraction map using the water Dixon image and the fat Dixon image. The modified fat fraction map is limited to fat fraction values that are greater than or equal to zero and less than or equal to one. When measuring the fat fraction within an organ of a healthy subject, in particular the liver, the fat signal is typically small and comparable to the noise level. Depending on how the fat fraction is calculated from the water signal and the fat signal, this can cause various voxels in the resulting fat fraction map to have negative values, which are of course physically impossible. The modified fat fraction map is limited to positive values. Using such a fat fraction map has the advantage that only physically possible values are displayed. The modified fat fraction map cannot be used to calculate the fat fraction value for an organ, however, because dropping the negative values introduces a bias and leads to too high aggregate fat fraction values.

An advantage of using the modified fat fraction map is that the fat fraction map can be displayed with a modified dynamic range. For example, if the negative values are removed then a color bar or a grey bar can be used for the physically possible values between 0 and 1 to show the fat fraction map with a greater degree of resolution. This may for example enable the operator of the medical system to better see anatomical structures in the modified fat fraction map.

A further advantage of using the modified fat fraction map is that the physician or medical professional looking at it is not immediately confronted with fat fraction values which are physically impossible. This may for example increase the acceptance of and the confidence in the fat fraction map. At the first look, the technician or physician would recognize that it is displaying non-physical values. This may have the effect that the subject or operator of the system is unable to use the fat fraction map displayed to select the region of interest.

The method further comprises displaying the modified fat fraction map on the user interface. The method further comprises receiving a selection of a measurement of ROI descriptive of an area within the common region of the subject from the user interface. In some instances, the measurement ROI would be selected using the modified fat fraction map. In other cases, other images such as the water and/or fat Dixon images or even one of the single echo images of the subject may be used. The method further comprises calculating fat fraction values for the measurement ROI using the water Dixon image and the fat Dixon image. As was mentioned above, determining the fat fraction value from the measurement ROI using the modified fat fraction map may result in a bias and a too high aggregate fat fraction value. In this step, the fat fraction value can be calculated in several ways. One way would be to have a pixel wise fat fraction map and use this for the calculation.

In other cases, the average water value for the measurement ROI can be calculated using the water Dixon image, the average fat value for the measurement ROI could be calculated using the fat Dixon image, and then these two average values for the measurement ROI could be used to perform the calculation of the fat fraction value. The method further comprises providing the fat fraction value. This may for example be displaying it on the user interface. In another example, the fat fraction value may be stored for later or archived with the Dixon images, for example stored together with the water Dixon image and the fat Dixon image in a DICOM file.

In another example, the method further comprises calculating a pixel wise fat fraction map from the water Dixon image and the fat Dixon image. A pixel wise fat fraction map as used herein encompasses a fat fraction map that is calculated pixel-by-pixel. For example, the water Dixon image and the fat Dixon image have corresponding voxels. The fat fraction is calculated for each individual pixel. For regions, such as the liver, that have a very low fat fraction, this means that the pixel wise fat fraction map may contain a large degree of noise.

As such, the pixel wise fat fraction map may have, in some cases, physically impossible values, such as negative values. The method further comprises displaying at least a portion of the pixel wise fat fraction map on the user interface in response to receiving the selection of the measurement ROI. The portion of the pixel wise fat fraction map comprises the measurement ROI. Providing the fat fraction value comprises displaying the fat fraction value. This example may be beneficial because examining the pixel wise fat fraction map may provide some information as to how well the measurement ROI was selected. For example, anatomical structures, such as blood vessels, may be more visible in the pixel wise fat fraction map.

In some examples the portion of the pixel wise fat fraction map is identical with the measurement ROI. For example, the user interface could superimpose the pixel wise fat fraction map on the measurement ROI itself and be limited to this. This may then provide the best of both worlds. For example, the modified fat fraction map is displayed with values bound to a lower limit of 0 and an upper limit of 1. Overall, the user may be able to better interpret the anatomical structures of the subject using the modified fat fraction map. The portion of the pixel wise fat fraction map as displayed may then be helpful in evaluating whether the proper measurement ROI was selected. This for example may provide a basis for providing an iterative method of determining the aggregate fat fraction value more accurately.

In another example, the fat fraction value is calculated by performing an average of the pixel wise fat fraction map within the measurement ROI. As was mentioned above, the pixel wise fat fraction map may contain a large amount of noise. However, if enough pixels are included in the measurement ROI then this noise will be reduced by the averaging.

In another example, the fat fraction value is calculated by determining an average fat value by averaging the fat Dixon value within the measurement ROI. The fat fraction value is further calculated by determining an average water value by averaging the water Dixon image within the measurement ROI. The fat fraction value is further determined by dividing the average fat value by the sum of the average water value and the average fat value. This example may be beneficial because it may provide for a very accurate determination of the fat fraction value.

In another example, the method further comprises repeatedly receiving a movement command for the measurement ROI from the user interface and repeatedly repositioning the measurement ROI using the movement command. Repositioning the measurement ROI comprises updating the displayed portion of the pixel wise fat fraction map. This example may be beneficial because it may provide for an iterative or adaptive method of determining the fat fraction value.

In another example, the method further comprises receiving a rating value by inputting a part of the pixel wise fat fraction map into an ROI rating module. The part of the pixel wise fat fraction map corresponds to the measurement ROI. The method further comprises displaying the rating value when displaying the fat fraction value. This example may be beneficial because it may provide for a means of choosing an ROI which provides for a better fat fraction value measurement.

In another example, the rating value is at least partially determined by creating a histogram of fat fraction values for the part of the pixel wise fat fraction value map and then fitting a statistical distribution to the histogram. The rating value is at least partially determined by applying a statistical test to measure the quality of the fit of the statistical distribution to the histogram. For example, in liver fat fraction measurements the pixel-by-pixel fat fractions typically have a Gaussian distribution. Measuring how closely a Gaussian distribution actually fits the histogram can then be used as an effective means of detecting the inclusion of undesired anatomical structures, such as blood vessels, in the measurement ROI.

In another example, the ROI rating module comprises a neural network configured to output a rating value in response to receiving the part of the pixel wise fat fraction map. This may be beneficial because it may provide for not just matching a distribution properly, but also for identifying anatomical structures directly in the image itself.

The neural network may be implemented in a variety of ways. In one example the neural network is implemented using a Convolutional Neural Network (CNN) with a regression head. CNNs are well-suited for image-based tasks due to their ability to capture spatial hierarchies and patterns. The architecture would typically include an input layer that accepts the MRI fat fraction map, several convolutional layers with ReLU activation for feature extraction, and pooling layers to reduce dimensionality while retaining important features. These would be followed by fully connected layers to interpret the features learned by the convolutional layers, and finally, a regression head with a single neuron to output the accuracy rating value.

In a second example, a U-Net architecture could be used to implement the neural network. U-Nets are very effective as they are able to take into account both global and local features, making it ideal for analyzing anatomical structures such as blood vessels that might affect the fat fraction measurement. The U-Net architecture consists of an encoder to capture context, a bottleneck as a bridge between the encoder and decoder, and a decoder to upsample and reconstruct spatial resolution. The output layer could be adapted for regression to predict a continuous value indicating accuracy.

In both examples, the training strategy could start with dataset preparation, including gathering a diverse dataset of MRI fat fraction maps with corresponding ground truth ratings or annotations. Preprocessing steps like normalizing the images, resizing them, and performing data augmentation such as rotation, translation, and scaling can enhance the model's robustness. The loss function used may be Mean Squared Error (MSE) to minimize the difference between predicted and true values, or a custom loss function that incorporates Gaussian fitting error or anatomical penalties to guide the network towards desired properties.

For the training process, optimizers like Adam or RMSprop can be used for efficient learning, along with learning rate scheduling techniques like decay or cyclic learning rates to possibly enable better convergence. Regular validation on a separate validation set may be used to monitor overfitting. Additionally, a mechanism to evaluate the Gaussian (or other statistical) distribution of voxel values may be implemented, and this information can be fed back into the network or the loss function to enhance performance. Handling anatomical structures can be achieved through segmentation preprocessing, using pre-trained models to mask or highlight blood vessels and other confounding factors, or incorporating attention mechanisms to help the model focus on relevant regions.

Evaluation metrics may include the Pearson Correlation Coefficient to measure the correlation between predicted ratings and ground truth, and R-Squared (Coefficient of Determination) to evaluate the proportion of variance captured by the model. Transfer learning can be leveraged to significantly reduce training time and improve accuracy, especially if the dataset is limited. Cross-validation techniques, such as k-fold cross-validation, should be implemented to ensure model generalizability and robustness.

In another example, the method further comprises logging the rating value as a function of the measurement ROI position as the movement commands are repeatedly received. The method further comprises displaying an indicator on the user interface to indicate a suggested movement direction using the logged rating values to optimize the rating value. The indicator may be implemented in several different ways. In one example, an indicator may be superimposed onto the circumference of the measurement ROI and different colors or greyscale values may be used to indicate in which direction a further extension of the measurement ROI likely improves or keeps or deteriorates the rating value. For example, traffic light colors (red/yellow/green) may be superimposed onto the circumference of the measurement ROI. Red may indicate a direction in which the rating value deteriorates. Yellow may indicate a direction in which the rating value stays the same. Green may indicate a direction in which the rating value improves.

In another example, the indicator may be an arrow which points in the direction of the area where the highest rating value was obtained. In other examples, a gradient between different rating values may be calculated and then these may be used to select a region which is suggested.

In another example, the method further comprises repeatedly rescaling a portion of the pixel wise fat fraction map to have a modified dynamic range within the measurement ROI in comparison to a dynamic range of the modified fat fraction map. The modified dynamic range preferably comprises negative fat fraction values. For example, the modified dynamic range could be restricted so that the voxels within the measurement ROI have a narrower range across the entire color bar or grey bar. This may enable the operator to see the anatomical details in greater detail. In another example, the dynamic range may cover the range of the true or unrestricted fat fraction values within the measurement ROI. This may for example be narrower than the range of the restricted fat fraction values in the entire map.

In another example, the portion of the pixel wise fat fraction map is an overlay limited to the measurement ROI. As was previously mentioned, this may be beneficial because it may provide the means to interactively see the anatomy as outlined by the modified fat fraction map, while at the same time seeing greater detail within the measurement ROI.

In another example, the method further comprises controlling a magnetic resonance imaging system with Dixon pulse sequence commands to acquire Dixon k-space data. The Dixon k-space data is descriptive of the common region of the subject. The method further comprises reconstructing the Dixon water image and the Dixon fat image from the Dixon k-space data. This example may be beneficial because the overall method may provide for an improved means of determining the fat fraction value.

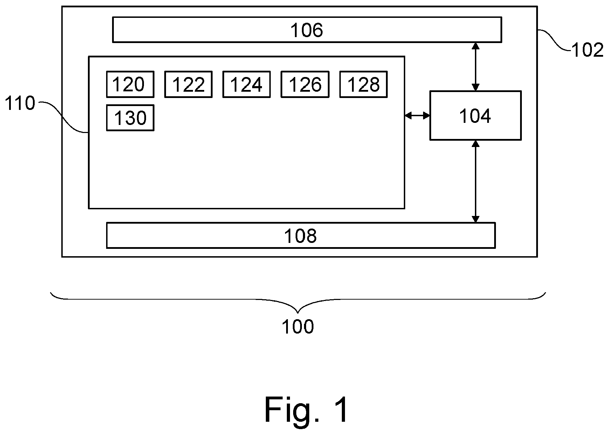

Fig. 1 illustrates an example of a medical system 100. The medical system 100 is shown as comprising a computer 102. The computer 102 may represent one or more computers or computing devices at one or more locations. The computer 102 is shown as comprising a computational system 104 that is in communication with an optional hardware interface 106, a user interface 108, and a memory 110. The computational system 104 may represent one or more computational systems or computational cores located at one or more locations. The hardware interface 106 may enable the computational system 104 to communicate with and/or control other components of the medical system 100 if they exist. The user interface 108 provides a means for an operator to interact with and control the operation and function of the medical system 100. The memory 110 is intended to represent various types of memory and storage that are accessible to the computational system 104. The memory may include volatile and non-volatile memory storage means and components. The memory in some embodiments may be based on or may rely on cloud-based data stored in logical pools across disparate, commodity storage servers located on premises or in a data center managed by a third-party cloud provider.

The memory 110 is shown as containing machine-executable instructions 120. The machine-executable instructions 120 may enable the computational system 104 to do such things as control other components of the medical system 100, perform numerical tasks, and perform image processing tasks. The machine-executable instructions 120 may also enable the computational system 104 to provide the user interface 108. The memory 110 is further shown as containing a water Dixon image 122 and a fat Dixon image 124 that depict a common region of a subject. The memory 110 is further shown as containing a modified fat fraction map 126 that is calculated pixel-by-pixel and which is limited to positive values, e.g., limited to values between 0 and 1.

The memory 110 is further shown as containing a measurement ROI 128, which is a region selected within the modified fat fraction map 126. The memory 110 is further shown as containing a fat fraction value 130 that was calculated for the measurement ROI 128 using the water Dixon image 122 and the fat Dixon image 124. The fat fraction value 130 may be calculated in a variety of different ways. For example, a water value can be calculated for the measurement ROI 128 using the water Dixon image 122 as well as a fat value for the measurement ROI 128 using the fat Dixon image 124. In other cases, the fat fraction value for individual voxels which make up the measurement ROI 128 can be calculated and then averaged to calculate the overall fat fraction value 130 for the measurement ROI 128.

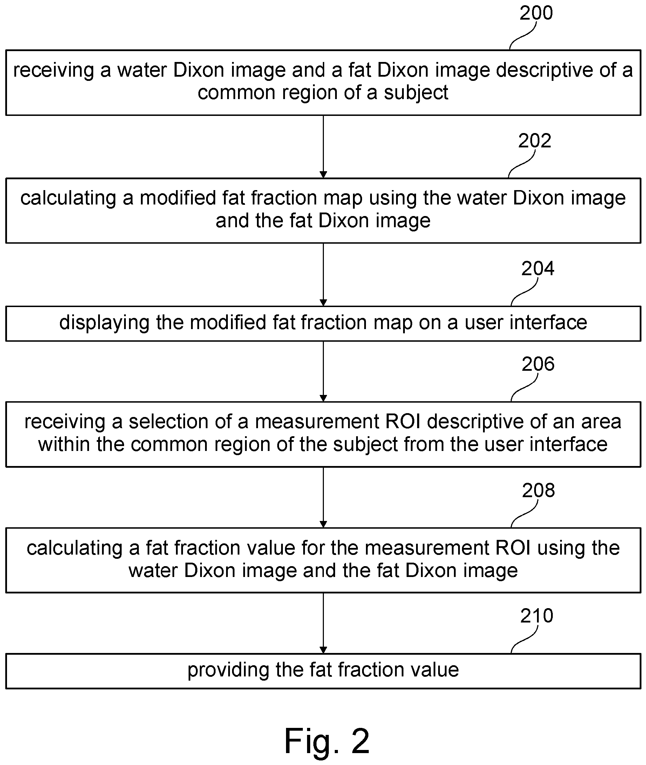

Fig. 2 shows a flowchart which illustrates a method of operating a medical system, for example the medical system 100 of Fig. 1. In step 200, the water Dixon image 122 and the fat Dixon image 124 are received. In step 202, the modified fat fraction map 126 is calculated using the water Dixon image 122 and the fat Dixon image 124. The modified fat fraction map 126 is limited to fat fraction values greater than or equal to 0 and less than or equal to 1. In step 204, the modified fat fraction map 126 is displayed on the user interface 108. In step 206, the selection of the measurement ROI 128 is received from the user interface 108. In step 208, the fat fraction value 130 is calculated for the measurement ROI 128 using the water Dixon image 122 and the fat Dixon image 124. In step 210, the fat fraction value 130 is provided. This may for example include displaying it on the user interface 108 as well as storing it in the memory 110. In some examples it could include appending the fat fraction value 130 to a DICOM file.

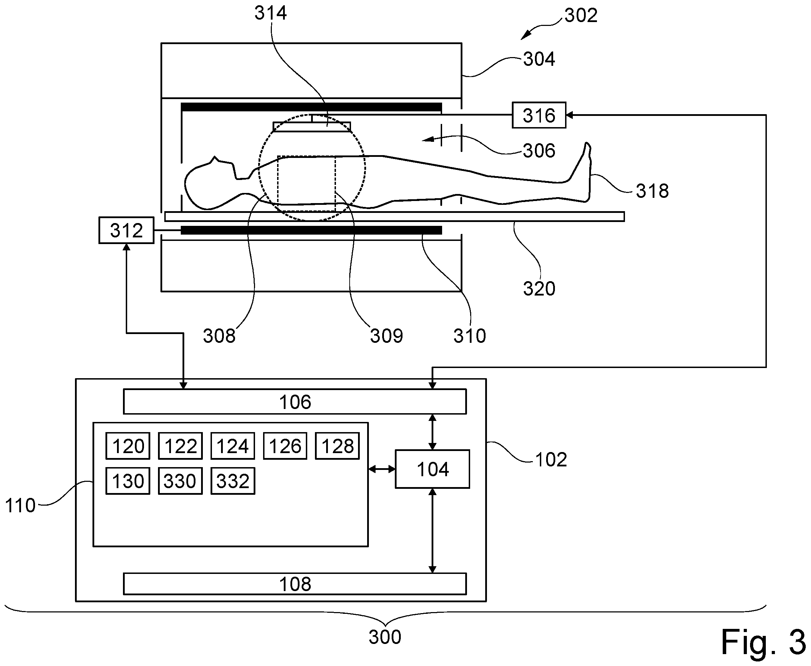

Fig. 3 illustrates a further example of a medical system 300. The medical system 300 is similar to the medical system 100 depicted in Fig. 1 except that it additionally comprises a magnetic resonance imaging system 302. The magnetic resonance imaging system 302 comprises a magnet 304. The magnet 304 is a superconducting cylindrical type of magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet. Such magnets may for instance be used in conjunction with charged particle beam therapy. An open magnet has two magnet sections, one above the other with a space in-between that is large enough to receive a subject. The arrangement of the two magnet sections is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data are acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310, which is used for the acquisition of k-space data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels. The transceiver 316 and the gradient coil power supply 312 are shown as being connected to the hardware interface 106 of the computer system 102.

The magnetic resonance imaging system 302 is shown as being controlled by the computer system 102. The memory 110 is further shown as containing Dixon pulse sequence commands 330 that are configured for controlling the magnetic resonance imaging system 302 to acquire k-space data 332 according to a Dixon magnetic resonance imaging protocol. The memory 110 is further shown as storing the k-space data 332 acquired by controlling the magnetic resonance imaging system 302 with the Dixon pulse sequence commands 330. The k-space data 332 can be reconstructed into the water Dixon image 122 and the fat Dixon image 124.

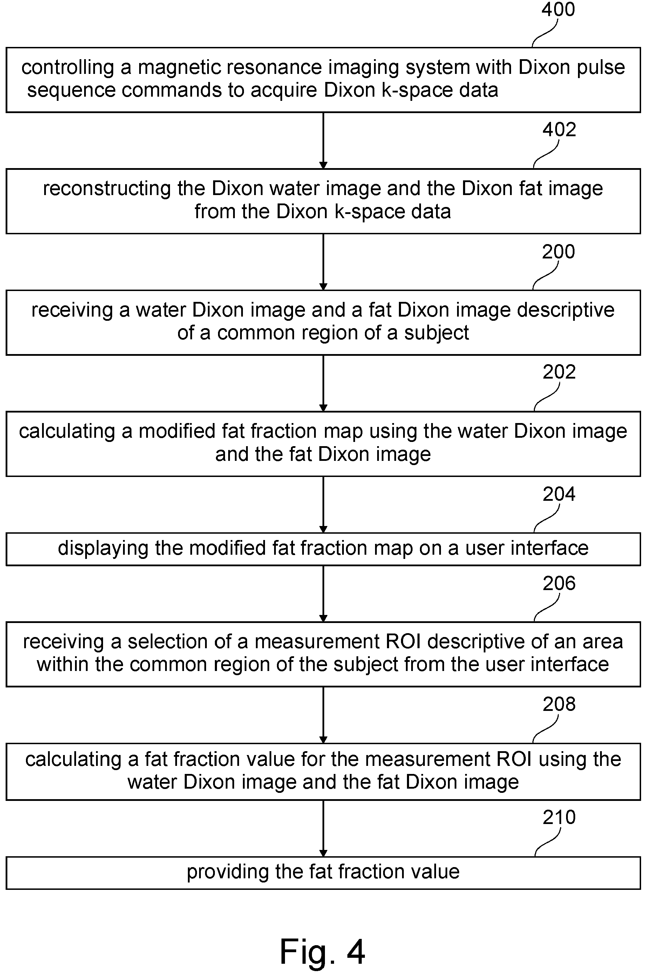

Fig. 4 shows a flowchart, which illustrates a method of operating a medical system, for example the medical system 300 of Fig. 3. In step 400 the magnetic resonance imaging system 302 is controlled with the Dixon pulse sequence commands 330 to acquire the Dixon k-space data 332. In step 402, the Dixon water image 122 and the fat Dixon image 124 are reconstructed from the Dixon k-space data 332. Steps 200, 202, 204, 206, 208, and 210 are performed similarly as illustrated in Fig. 2.

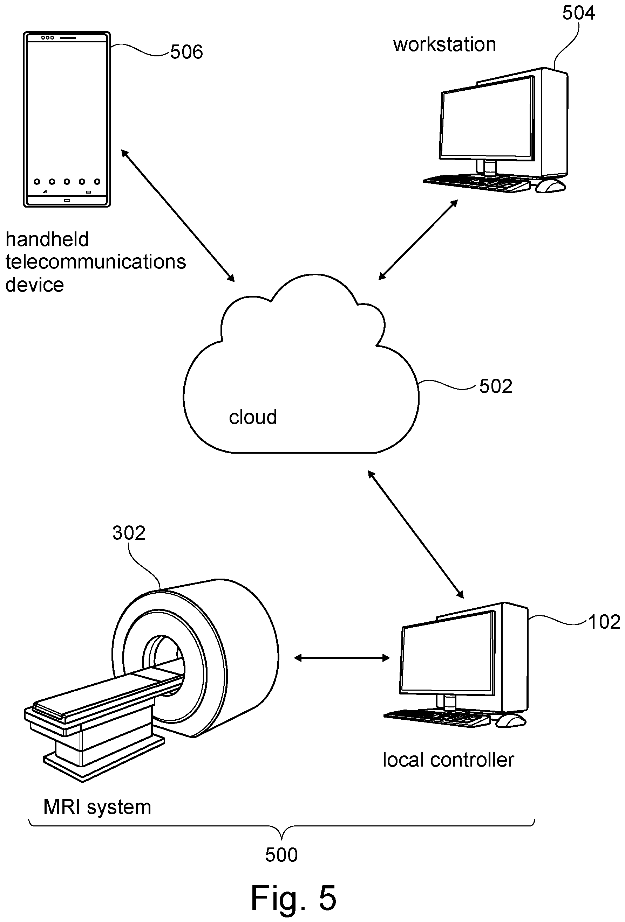

Fig. 5 illustrates a further example of a medical system 500. The medical system 500 comprises the magnetic resonance imaging system 302, a local controller 102, as well as a cloud-based computing system 502. The medical system 500 may further comprise any or both of a radiology workstation 504 and a handheld telecommunications device 506,. The functionality of the computer 102 in Figs. 1 and 3 can be distributed amongst the various devices 102, 502, 504, and 506 in Fig. 5. The cloud-based computing system 500 could be made up of networked servers and/or virtual machines available over the internet or other network.

As an example, different means of calculating the fat faction value are investigated. Three different approaches are considered to derive signal, such as water and fat signal, from the composite signal $S_n$ measured at echo times $t_n$.

The conventional, first approach estimates complex water ($W$) and fat ($F$) signal based on the signal model:

$$S_n = (W + c_n F)\, \mathrm{e}^{(-R_2{}^* + \mathrm{i}2\pi f)t_n}, \qquad (1)$$

where the complex c describes the amplitude and phase modulation of $F$ over time due to the spectral composition of fat, and the real $R_2{}^*$ and $f$ denote the effective transverse relaxation rate and off-resonance frequency, respectively.

The second approach estimates complex water and in-phase ($IP$) signal based on the slightly modified signal model:

$$S_n = \left((1 - c_n)W + c_n IP\right) \mathrm{e}^{(-R_2^* + \mathrm{i}2\pi f)t_n}. \qquad (2)$$

Assuming the phase ($p$) of the complex water and fat signal to be equal at $t = 0$, the third approach estimates real water ($W_R$) and fat ($F_R$) signal, along with $p$, based on the signal model:

$$S_n = (W_R + c_n F_R)\, \mathrm{e}^{\mathrm{i}p + (-R_2^* + \mathrm{i}2\pi f)t_n}. \qquad (3)$$

Six different calculations of the fat fraction value ($FF$) from the signal derived with these three approaches are considered.

Based on the first signal model, the first calculation provides a complex $FF$:

$$FF_1 = F/(W + F), \qquad (4)$$

whereas the second calculation provides a real non-negative $FF$:

$$FF_2 = |F|/(|W| + |F|), \qquad (5)$$

which is identical to:

$$FF_2 = 1 - |W|/(|W| + |F|). \qquad (6)$$

The third and fourth calculations provide a real $FF$:

$$FF_3 = |F|/(|W + F|) \qquad (7)$$

$$FF_4 = 1 - |W|/(|W + F|). \qquad (8)$$

where $FF_3$ is non-negative, while $FF_4$ is not.

Based on the second signal model, the fifth calculation provides a real $FF$:

$$FF_5 = 1 - |W|/(|IP|), \qquad (9)$$

as does the sixth calculation, based on the third signal model:

$$FF_6 = F_R/(W_R + F_R), \qquad (10)$$

which is identical to:

$$FF_6 = 1 - W_R/(W_R + F_R). \qquad (11)$$

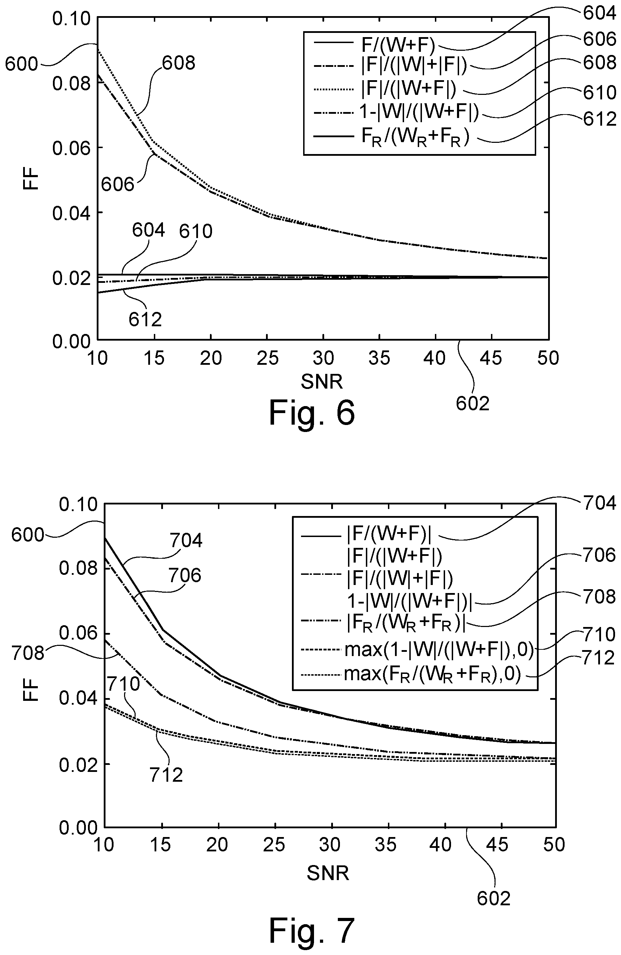

These six calculations were compared in simulations in Fig. 6, which illustrates the effect of noise on the fat fraction value calculated using different equations. In the graph the fat fraction is the y-axis 600 and the signal-to-noise ratio (SNR) is the x-axis 602. $FF_1$ is labeled 604. $FF_2$ is labeled 606. $FF_3$ is labeled 608. $FF_4$ is labeled 610, and $FF_6$ is labeled 612.

In Fig. 6, representative results are plotted for a nominal $FF$ of 2% with varying levels of SNR (602). $FF_5$ is not explicitly shown, since it closely matched $FF_4$. $S$ was synthesized according to Eq. (1), choosing $t_n = 0.97$ ms + $(n$-1)*0.71 ms with $n = 1 \ldots 6$, $R_2^* = 66.7$ s$^{-1}$, $f = 20$ Hz, and a seven-peak spectral model of fat. 10.000 times one complex noise sample with Gaussian distribution was generated per echo, scaled according to the target SNR, and added to $S$.

Only $FF_2$ and $FF_3$ in this graph show a considerable noise bias at lower SNR. The noise bias is removed in $FF_4$, $FF_5$, and $FF_6$. Notably, the first calculation, providing a complex $FF$, performs even better in this regard. However, it, as well as the fourth, fifth, and sixth calculation, all rely on an averaging of the complex or real $FF$ values. The calculations $FF_1$, $FF_4$, $FF_5$, and $FF_6$ may be used to calculate the pixel wise fat fraction map.

Fig. 7 shows similar measurements to those shown in Fig. 6. However, the various fat fraction calculation methods have been modified so that they are limited to positive values. The modified $FF_1$ is labeled 704. It closely matches $FF_3$. A modified $FF_4$ is labeled 706. It closely matches a modified $FF_6$, which is labeled 708. Instead of taking the absolute value of a potentially negative value, it is also possible to take the maximum of the potentially negative value and 0. This leads to another modified $FF_4$, which is labeled 710, and another modified $FF_6$, which is labeled 712.

It is an insight of the present invention that considerable noise bias at lower SNR reappears in $FF_1$, $FF_4$, $FF_5$, and $FF_6$ if the complex or real $FF$ values are restrained to real non-negative $FF$ values, as evident from Fig. 7. Any of the modified FF calculations, as illustrated in Fig. 7, may be used to calculate the modified fat fraction map.

Only modifications to limit $FF$ values to positive values have been introduced so far. However, it is straightforward to introduce similar modifications to limit $FF$ values to values of 1 or less.

It is a further insight of the present invention that the primary difference between $FF_4$ and $FF_5$ on the one hand and $FF_2$ and $FF_3$ on the other hand is that real negative $FF$ values occur, in which case $|W|$ is larger than $|W+F|$ or $|IP|$ and $W$ and $F$ are rather out-of-phase than in-phase.

It is an even further insight of the present invention that the sixth calculation also provides real negative $FF$ values and does not ensure that $W$ and $F$ are in-phase, because if either $W$ or $F$ becomes negative, they are actually opposed-phase.

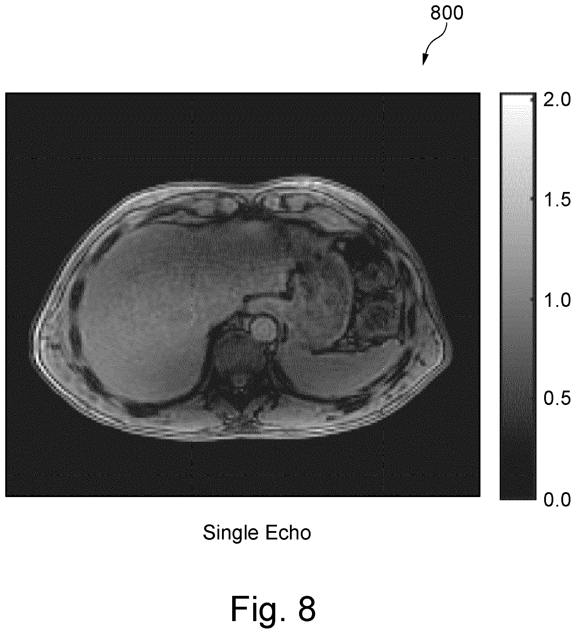

Fig. 8-12 illustrate various magnetic resonance images and fat fraction maps calculated from a single set of Dixon k-space data 332. Fig. 8 shows a single echo image reconstructed from the Dixon k-space data 332. This shows the various internal anatomy of the subject 318 in detail. In some examples, the single echo magnetic resonance image 800 may be used to select the measurement ROI 128.

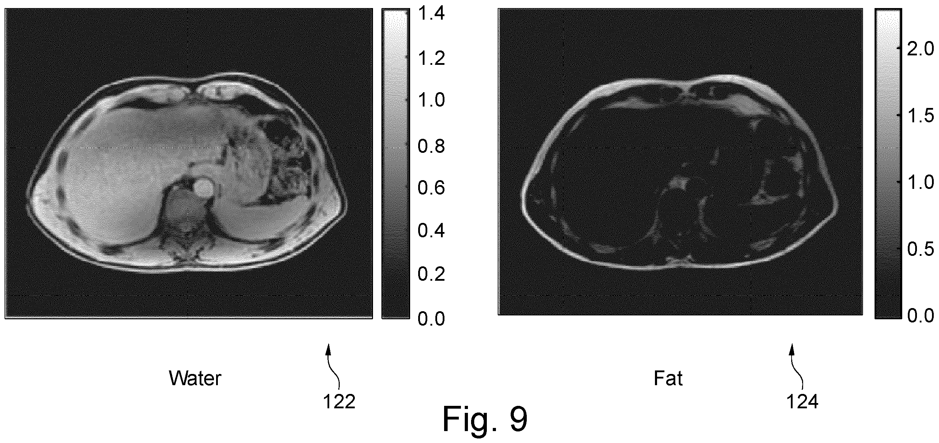

Fig. 9 illustrates the water Dixon image 122 and the fat Dixon image 124 from the same Dixon k-space data 332. It can be seen that both images 122 and 124 depict the common region of the subject. In this case the common region is the field of view 309.

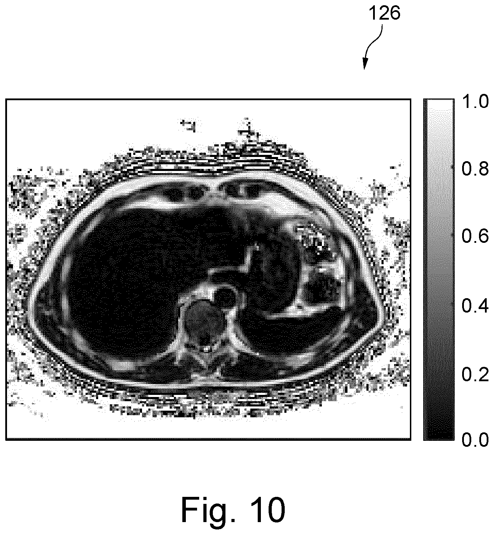

Fig. 10 illustrates a modified fat fraction map 126 that was calculated from the Dixon k-space data 332. It can be seen that the fat fraction values are limited between 0 and 1. In regions such as the liver, this may result in a bias which shifts the fat fraction value to a larger value if the individual voxels within a measurement ROI are averaged. However, no physically impossible values are displayed, which would be likely to confuse a medical professional or radiologist.

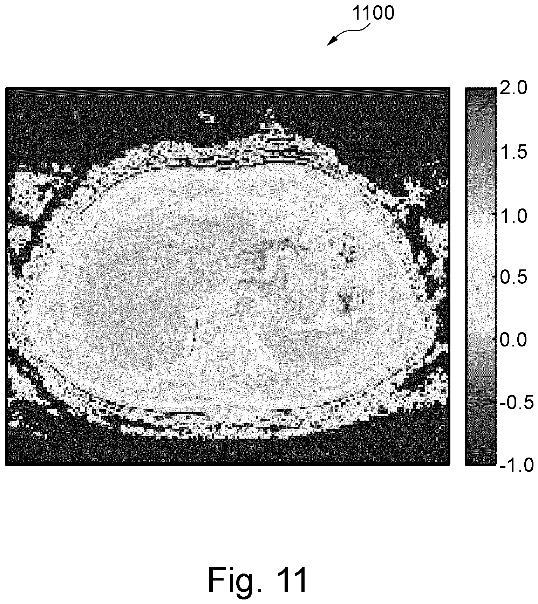

Fig. 11 illustrates a pixel wise fat fraction map 1100. It can be seen that some of the values here are less than 0 and some of the values are greater than 1. The map clearly displays some physically impossible values which are due to noise in the Dixon k-space data 332. In comparing Figs. 10 and 11, the map in Fig. 10 shows a reduced dynamic range which better depicts the anatomy and enables a better selection of the measurement ROI 128.

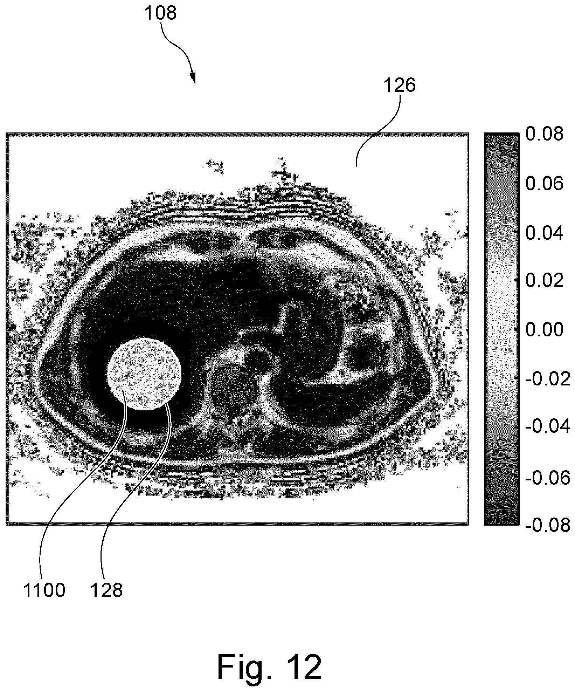

Fig. 12 illustrates an example of a user interface 108. The user interface shows a hybrid image. In the background is the modified fat fraction map 126 which details the anatomy of the subject such that only physically possible values are displayed. On this, the measurement ROI 128 has already been selected and the portion of the pixel wise fat fraction map 1100 corresponding to the measurement ROI 128 has been superimposed upon it. In this case the physician can look at the individual values within the measurement ROI 128 and assess if there is some anatomical structure, such as a blood vessel, or an artifact, such as a ghost from respiratory motion, which may confound the measurement. The user interface 108 may enable the operator to select a better measurement ROI 128 and therefore obtain a more accurate measurement of the fat fraction value 130.

Although in Fig. 12 the pixel wise fat fraction map 1100 corresponding to the measurement ROI 128 has been superimposed on the modified fat fraction map 126, other alternatives are contemplated, such as displaying side by side the modified fat fraction map 126 corresponding to the ROI 128 and the pixel wise fat fraction map 1100 corresponding to the measurement ROI 128, displaying side by side the modified fat fraction map 126 corresponding and the pixel wise fat fraction map 1100 wherein for both the ROI is delimited for comparison purpose.

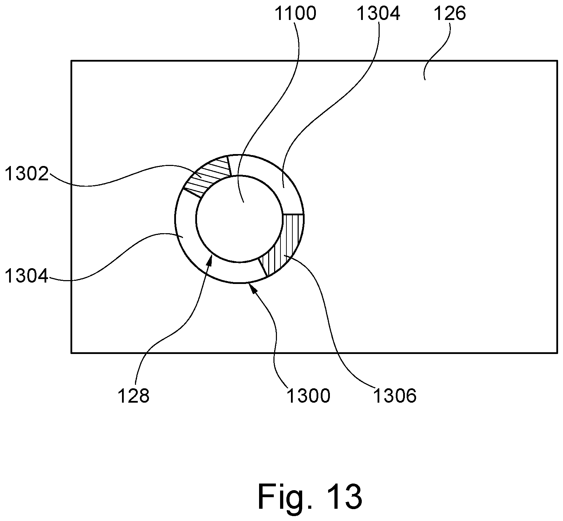

Fig. 13 depicts a modification of the user interface 108 shown in Fig. 12. In this example, there is an indicator 1300 which is formed around a boundary of the measurement ROI 128. The user interface is divided into several regions 1302, 1304, and 1306. In this example movement of the measurement ROI in the direction of region 1302 would cause a worsening of the rating value. Movement in the direction of region 1304 would cause the rating value to remain the same or within a predetermined range of the current rating value. Movement in the direction of region 1306 would cause an improvement in the rating value. This may provide a means of guiding an operator to select an optimal or preferred location for the measurement ROI.

Various colors, greyscales, or patterns may be assigned to the different regions 1302, 1304, and 1306. In one example, red could be assigned to region 1302, yellow could be assigned to region 1304, and green could be assigned to region 1306.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational systems to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the C programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, WLAN connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, television screen, touch screen, tactile electronic display, Braille screen, Cathode Ray Tube (CRT), storage tube, bi-stable display, electronic paper, vector display, flat panel display, Vacuum Fluorescent (VD) display, Light-Emitting Diode (LED) displays, Electro-Luminescent Display (ELD), Plasma Display Panels (PDP), Liquid Crystal Display (LCD), Organic Light-Emitting Diode (OLED) displays, a projector, and head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1.  A method of determining a fat fraction value (130), wherein the method comprises:

    - receiving (200) a water Dixon image (122) and a fat Dixon image (124) descriptive of a region of a subject (318);
    - calculating (202) a modified fat fraction map (126) using the water Dixon image and the fat Dixon image, wherein the modified fat fraction map is limited to fat fraction values greater than or equal to zero;
    - displaying (204) the modified fat fraction map on a user interface (108);
    - receiving (206) a selection of a measurement region of interest (128), ROI, descriptive of an area within the region of the subject from the user interface;
    - calculating (208) the fat fraction value for the measurement ROI using the water Dixon image and the fat Dixon image; and
    - providing (210) the fat fraction value.

2.  The method of claim 1, wherein the modified fat fraction map is limited to fat fraction values greater than or equal to zero and less than or equal to one;

3.  The method of claim 1 or 2, wherein the method further comprises:

    - calculating a pixel wise fat fraction map (1100) from the water Dixon image and the fat Dixon image; and
    - displaying at least a portion of the pixel wise fat fraction map on the user interface in response to receiving the selection of the measurement ROI, wherein the portion of the pixel wise fat fraction map comprises the measurement ROI; wherein providing the fat fraction value comprises displaying the fat fraction value.

4.  The method of claim 3, wherein the fat fraction value is calculated by performing an average of the pixel wise fat fraction map within the measurement ROI.

5.  The method of claim 3, wherein the fat fraction value is calculated by:

    - determining an average fat value by averaging the fat Dixon image within the measurement ROI;
    - determining an average water value by averaging the water Dixon image within the measurement ROI; and
    - calculating the fat fraction by dividing the average fat value by the sum of the average water value and the average fat value.

6.  The method of any of the claims 3 to 5, wherein the method further comprises:

    - receiving a movement command for the measurement ROI from the user interface; and
    - repositioning the measurement ROI using the movement command, wherein repositioning the measurement ROI comprises updating the displayed portion of the pixel wise fat fraction map.

7.  The method of claim 6, wherein the method further comprises:

    - receiving a rating value by inputting a part of the pixel wise fat fraction map into a ROI rating module, wherein the part of the pixel wise fat fraction map corresponds to the measurement ROI; and
    - displaying the rating value when displaying the fat fraction value.

8. The method of claim 7, wherein the rating value is at least partially determined by:

   - creating a histogram of fat fraction values for the part of the pixel wise fat fraction map;
   - fitting a statistical distribution to the histogram, wherein the statistical distribution is preferably a Gaussian distribution; and
   - assigning the rating value by applying a statistical test to measure the fitting of the Gaussian distribution to the histogram.

9. The method of claim 8, wherein the ROI rating module comprises a neural network configured to output the rating value in response to receiving the part of the pixel wise fat fraction map.

10. The method of any of claims 7 to 9, wherein the method further comprises:

    - logging the rating value as a function of measurement ROI position as the movement commands are repeatedly received;
    - displaying an indicator (1300) on the user interface to indicate a suggested movement direction using the logged rating values to optimize the rating value.

11. The method of any of claims 3 to 10, wherein the method further comprises repeatedly rescaling the portion of the pixel wise fat fraction map to have a modified dynamic range within the measurement ROI in comparison to a dynamic range of the modified fat fraction map, and wherein the modified dynamic range preferably comprises negative fat fraction values.

12. The method of any of claims 2 to 11, wherein the portion of the pixel wise fat fraction map is an overlay limited to the measurement ROI.

13. The method of any one of the preceding claims, wherein the method further comprises:

    - controlling (400) a magnetic resonance imaging system (302) with Dixon pulse sequence commands (330) to acquire Dixon k-space data, wherein the Dixon k-space data is descriptive of the region of the subject; and
    - reconstructing (402) the Dixon water image and the Dixon fat image from the Dixon k-space data.

14. A computer program product comprising machine executable instructions (120) configured for causing a computational system (104) to perform the method of any of claims 1 to 13.

15. A medical system (100, 300, 500) comprising:

    - a computational system (104), configured to perform the method of any of claims 1 to 13.

Fig. 1

200

receiving a water Dixon image and a fat Dixon image descriptive of a common region of a subject

202

calculating a modified fat fraction map using the water Dixon image and the fat Dixon image

204

displaying the modified fat fraction map on a user interface

206

receiving a selection of a measurement ROI descriptive of an area within the common region of the subject from the user interface

208

calculating a fat fraction value for the measurement ROI using the water Dixon image and the fat Dixon image

210

providing the fat fraction value

Fig. 2

Fig. 3

EP 4 711 792 A1

400

controlling a magnetic resonance imaging system with Dixon pulse sequence commands to acquire Dixon k-space data

402

reconstructing the Dixon water image and the Dixon fat image from the Dixon k-space data

200

receiving a water Dixon image and a fat Dixon image descriptive of a common region of a subject

202

calculating a modified fat fraction map using the water Dixon image and the fat Dixon image

204

displaying the modified fat fraction map on a user interface

206

receiving a selection of a measurement ROI descriptive of an area within the common region of the subject from the user interface

208

calculating a fat fraction value for the measurement ROI using the water Dixon image and the fat Dixon image

210

providing the fat fraction value

Fig. 4

handheld telecommunications device — 506

workstation — 504

cloud — 502

302

local controller — 102

MRI system

500

Fig. 5

Fig. 6

Fig. 7

800

Single Echo

# Fig. 8

Water

122

Fat

124

Fig. 9

126

Fig. 10

1100

Fig. 11

Fig. 12

**Fig. 13**

Europäisches
Patentamt
European
Patent Office
Office européen
des brevets

# EUROPEAN SEARCH REPORT

**Application Number**

EP 24 20 0222

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOUCHUN HARRY HU: "Magnetic resonance techniques for fat quantification in obesity", SIGNAL&INFORMATION PROCESSING ASSOCIATION ANNUAL SUMMIT AND CONFERENCE (APSIPA ASC), 2012 ASIA-PACIFIC, IEEE, 3 December 2012 (2012-12-03), pages 1-10, XP032309910, ISBN: 978-1-4673-4863-8 * page 5 - page 6; figures 7, 8 *<br>----- | 1-15 | INV.<br>G01R33/48<br>A61B5/00<br>G01R33/56<br><br>ADD.<br>G01R33/54 |
| X | US 2005/215882 A1 (CHENEVERT THOMAS L [US] ET AL) 29 September 2005 (2005-09-29) * paragraphs [0048] - [0054], [0058], [0059], [0069]; figure 4 *<br>----- | 1-15 | |
| A | US 2023/386035 A1 (WANG CHUN-YU [CN] ET AL) 30 November 2023 (2023-11-30) * paragraph [0076] - paragraph [0106]; figure 7 *<br>----- | 6-8,10 | |
| | | | TECHNICAL FIELDS SEARCHED (IPC)<br><br>G01R<br>A61B |

The present search report has been drawn up for all claims

| Place of search | Date of completion of the search | Examiner |
|---|---|---|
| Munich | 19 February 2025 | Raguin, Guy |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 20 0222

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2025

| Patent document cited in search report | Publication date | Patent family member(s) | | Publication date |
|---|---|---|---|---|
| US 2005215882 A1 | 29-09-2005 | NONE | | |
| US 2023386035 A1 | 30-11-2023 | CN | 117173076 A | 05-12-2023 |
| | | US | 2023386035 A1 | 30-11-2023 |

EPO FORM P0459

For more details about this annex : see Official Journal of the European Patent Office, No. 12/82